# FASCICULE DE BREVET EUROPEEN

(11) **EP 2 473 125 B1**
(45) Date de publication et mention de la délivrance du brevet: **27.02.2019**
(21) Numéro de dépôt: 10762946.1
(22) Date de dépôt: 27.08.2010
(51) Int. Cl.: A61B 17/34, A61B 8/08, A61B 34/10

(54) **SYSTÈME DE CONTRÔLE POUR LE GUIDAGE PRÉCIS VERS LA PROSTATE D'UNE AIGUILLE PERCUTANÉE**
STEUERSYSTEM ZUR PRÄZISEN LENKUNG EINER PERKUTANEN NADEL ZUR PROSTATA
CONTROL SYSTEM FOR PRECISELY GUIDING A PERCUTANEOUS NEEDLE TOWARD THE PROSTATE

(30) Priorité: 31.08.2009 FR 0904124
(43) Date de publication de la demande: 11.07.2012
(73) Titulaire: Koelis, 38100 Grenoble (FR); Université Grenoble Alpes, 38400 Saint-Martin-d'Hères (FR)
(72) Inventeur: BAUMAN, Michaël, F-38000 Grenoble (FR); HUNGR, Nikolaï, F-38700 Grenoble (FR); LEROY, Antoine, F-38240 Meylan (FR); TROCCAZ, Jocelyne, F-38320 Eybens (FR); DAANEN, Vincent, F-38134 Saint Joseph de Rivière (FR)
(74) Mandataire: Novagraaf Technologies
(86) Numéro de dépôt international: PCT/FR2010/000587
(87) Numéro de publication internationale: WO 2011/023866

(56) Documents cités:
- EP-A1- 1 088 524
- WO-A1-98/31273
- WO-A1-99/60921
- US-A1- 2003 018 232
- US-A1- 2007 016 067

## Description

### Domaine de l'invention

La présente invention concerne le domaine des équipements de traitement et de diagnostic des pathologies prostatiques.

Ces équipements sont destinés à commander le déplacement d'une aiguille et son insertion dans la prostate selon une direction et une profondeur déterminées.

Le traitement du cancer de la prostate vise à guérir le patient par la destruction des tissus malades en préservant les tissus sains environnants. Il est en conséquence important de pouvoir délivrer les agents thérapeutiques avec précision aux tissus cancéreux. Dans le cas où l'agent est placé avec une précision insuffisante, il est possible qu'il agisse sur des tissus sains, et potentiellement sur des structures nobles telles que la vessie, le rectum, ou l'urètre. Dans ces cas, le patient risque de souffrir de pathologies telles que l'impuissance ou l'incontinence. Le taux d'effets secondaires encore élevé pour les traitements minimalement invasifs du cancer de la prostate peut être expliqué par le fait que les traitements actuels souffrent d'une double défaillance : 1) Les outils de délivrance des agents thérapeutiques (par exemple une aiguille de ponction contenant un grain radioactif dans le cadre d'une curiethérapie) peuvent être placés loin de la cible à cause de facteurs tels que la mobilité de la prostate ou la flexion de l'aiguille, et 2) ces écarts ne sont pas détectés préalablement à la délivrance de l'agent.

Pour certains traitements, une délivrance très précise des agents thérapeutiques permet de couvrir la totalité des tissus à traiter de manière régulière et avec une dose ou un effet cumulatifs suffisants (curiethérapie, cryothérapie, HIFU par exemple). Le risque de récidive, lié au sous-traitement des zones cancéreuses, dépend donc fortement de la précision de mise en oeuvre de la thérapie.

En somme, jusqu'à présent les traitements prostatiques sont conduits de manière globale, sur l'ensemble de la glande. Les raisons principales sont l'incertitude de localisation des tissus cancéreux avec les moyens diagnostiques actuels et le caractère multifocal du cancer prostatique. La troisième raison est le manque de précision des dispositifs de traitement actuels, le risque de manquer ou de sous-doser une cible localisée étant relativement élevé.

Le diagnostic du cancer de la prostate est affecté par le même problème de manque de précision dû à l'imprécision des dispositifs et le manque de gestion de l'environnement mou et mobile de la prostate. Une meilleure précision est nécessaire pour pouvoir apporter un diagnostic plus fiable et plus complet, avec notamment une information de localisation des zones cancéreuses, qui pourrait éventuellement aussi servir à planifier et guider des traitements focalisés.

En conclusion il y a donc un besoin fort de pouvoir guider des outils diagnostiques et des agents thérapeutiques avec un maximum de précision au foyer cancéreux, afin d'améliorer la fiabilité du diagnostic, et de minimiser les effets secondaires, les récidives et la morbidité associée du traitement. Il est également souhaitable d'améliorer la détection des situations où le dispositif de délivrance de l'agent n'a pas atteint la cible, et ceci avant de délivrer l'agent.

### Etat de la technique

Dans ce contexte de traitement et diagnostic précis du cancer de la prostate, on a proposé, dans l'état de la technique, différentes solutions permettant d'améliorer la traçabilité sur l'organe des zones de pénétration de l'aiguille ainsi que la localisation des zones cancéreuses.

Une première solution décrite dans le brevet international WO2009071766 concerne un procédé d'imagerie prostatique comportant des étapes d'enregistrement et de traitement d'images acquises par la tête échographique d'une sonde rectale équipée d'un guide de ponction actif. Ce procédé comporte des étapes de traitement d'une partie au moins des images acquises par la sonde pour calculer des transformations vers un référentiel image de référence lié à la position initiale de la prostate, une partie au moins des images acquises pendant les ponctions successives comprenant la localisation des différentes positions de l'aiguille étant enregistrée en vue d'une visualisation de leurs représentations sur une image unique comprenant une partie au moins de la prostate. Ce brevet de l'art antérieur concerne également un système pour l'imagerie prostatique mettant en oeuvre ledit procédé d'imagerie prostatique.

Cette solution améliore l'acquisition d'une information graphique sur la prostate, et permet la mise en concordance de différentes images de la prostate, acquises avec des systèmes d'imagerie complémentaires ainsi que par une sonde rectale solidaire de l'aiguille de traitement. Elle permet donc d'améliorer le guidage et la localisation de l'aiguille par rapport à des zones d'intérêt de la prostate.

Ce système d'imagerie est utilisable tant pour des équipements manipulés par un chirurgien de façon manuelle, que par des équipements robotisés.

On connaît également dans l'état de la technique des méthodes de contrôle, parfois reliés à des équipements robotisés pour la réalisation de biopsies et de traitements prostatiques.

L'état de la technique le plus proche est constitué par le de brevet américain US20070270687 qui décrit une méthode de recalage entre images échographiques 3D pour le suivi du mouvement d'un outil dans le référentiel image en relevant les différences entre les deux images par soustraction. Une application proposée dans ce document de l'art antérieur est le suivi du mouvement de l'extrémité d'une aiguille de curiethérapie pour permettre une dosimétrie adaptative et palier les erreurs de positionnement de l'aiguille.

Un autre état de la technique proche à la présente invention consiste du brevet canadien CA2654344 qui décrit un équipement et une méthode pour le positionnement et l'insertion d'une aiguille autour d'un point fixe (remote center of motion). Selon une application décrite dans ce document, ces équipement et technique peuvent être utilisés pour le positionnement d'une aiguille de biopsie, en comparant visuellement des images 3D échographiques pour vérifier le mouvement ou le gonflement de la prostate après retrait de l'aiguille.

Encore un autre état de la technique proche à la présente invention consiste du brevet américain US6423009 qui décrit un système pour le guidage d'outils percutanés pour la thérapie de la prostate utilisant des images 3D échographiques. Un exemple d'utilisation de la technique décrit le positionnement de sondes de cryothérapie dans la cryothérapie de la prostate. Avec cet exemple est décrite une méthode de positionnement des sondes avec une vérification du bon positionnement des aiguilles par rapport au planning.

La demande de brevet internationale WO 2007085953 consiste d'encore un autre document de l'art antérieur. Il décrit un équipement et une méthode pour l'insertion d'une aiguille selon une trajectoire prédéterminée et à une profondeur prédéterminée, afin d'atteindre une zone cible des tissus d'un organe mou, avec un contrôle par imagerie ultrasonore. Les applications proposées dans ce document de l'art antérieur sont la biopsie de la prostate ou la curiethérapie de la prostate. La méthode met en oeuvre une zone d'approche conique à travers la paroi périnéale. L'aiguille traverse la peau du patient à travers un point pivot. L'orientation du support de l'aiguille est assurée par des joints sphériques.

Le brevet américain US6846282B décrit un moyen de curiethérapie réalisé à l'aide d'une aiguille pour l'injection d'un produit radioactif dans un organe tel que la prostate. Des marqueurs fixés sur le corps du patient définissent un système de coordonnées permettant d'analyse en temps réel le déplacement de l'aiguille dans le référentiel du patient. Une solution alternative consiste à utiliser des repères anatomiques.

Le brevet EP1088524 décrit de manière générale un système et un procédé de mise en oeuvre de curiethérapie de la prostate basé sur une image échographique reconstruite en 3D, un planning pré-opératoire d'insertion d'aiguilles comprenant une segmentation des organes environnants et une évaluation per-opératoire du placement et de la déflection des aiguilles par rapport au planning. Il n'est pas fait mention d'une quantification tridimensionnelle du bougé de la prostate dans cette invention.

Le brevet US2003/0018232 présente un système automatisé d'implantation de grains radioactifs. Le principe de positionner une aiguille en largeur, hauteur et profondeur dans l'organe, afin d'y insérer un grain radioactif. Le système est étalonné par rapport à l'organe (la prostate) par l'ajustement d'un plan de base par rapport à l'organe vu sous échographie. La sonde d'échographie est solidaire du robot et mobilisée selon un axe parallèle à l'axe d'insertion des aiguilles. Au cours de la procédure d'insertion, une acquisition échographique plan par plan de l'organe peut être ordonnée afin d'effectuer un recalage et d'adapter en conséquence le plan de base d'insertion des aiguilles. De même que dans le brevet EP1088524, le bougé 3D de la prostate au cours de l'insertion d'une aiguille ne peut pas être pris en compte pour une compensation automatique et précise du planning par rapport à l'organe.

Le brevet US2007/0016067, s'il décrit un système et un procédé robotisé de ponction sous imagerie per-opératoire, ne tient pas compte dans sa boucle de contrôle de la position relative de l'outil de ponction avec l'organe visé.

### Problème technique

Comme décrit ci-dessus, les solutions connues ne sont pas totalement satisfaisantes, car la prostate est un organe déformable et mobile. Les solutions de l'art antérieur sont de ce fait perturbées par les modifications de l'organe et ne permettent pas une localisation réellement précise. La présente invention s'appuie sur une quantification automatique précise du bougé de la prostate effectuée à partir d'une comparaison entre une image 3D instantanée de la prostate après insertion d'une aiguille et une image 3D de référence. L'invention tire avantage de l'échographie 3D instantanée qui au contraire de l'échographie 3D reconstruite génère en de manière instantanée un volume dense et cohérent, sans mobiliser ni la sonde ni l'organe.

Les bougés de la prostate, consistant à la fois dans les déplacements et les déformations, résultent en effet de différents phénomènes :
- Sous la pression d'une aiguille de ponction ou de traitement, la prostate se déforme et se déplace à l'intérieur du corps humain.
- Les ponctions provoquent des lésions induisant un gonflement de la prostate, au cours de l'intervention. Les mouvements du patient, notamment la respiration, produisent également des déformations et des déplacements de la prostate.
- Pour obtenir une image de bonne qualité, la sonde rectale doit venir en contact avec la paroi rectale proche de la prostate et exercer une légère pression produisant également une déformation et un déplacement de la prostate.

Les solutions de l'art antérieur ne prennent en compte les bougés de la prostate ni de manière automatique ni durant le temps de l'insertion de l'aiguille. De plus cette évaluation des bougés n'est pas utilisée dans le but d'adapter le guidage de l'aiguille dans le référentiel prostatique au cours de son insertion.

### Solution apportée par l'invention.

Le but de l'invention est de remédier à ces inconvénients en proposant un équipement permettant de pallier les bougés de la prostate d'une manière automatique et d'adapter le guidage de l'aiguille au cours de son insertion, afin d'améliorer la précision des prélèvements ou traitements localisés de la prostate.

L'invention permet également d'améliorer la répartition de la dose de traitement et la protection des tissus sains et des structures critiques avoisinantes par une prise en compte plus précise de la configuration et la position de la prostate au moment du traitement.

A cet effet, l'invention concerne selon son acception la plus générale un équipement porte-aiguille pour un traitement de la prostate impliquant une succession d'étapes d'insertion d'une aiguille dans la prostate, comportant une sonde rectale d'imagerie 3D ultrasonore et un calculateur pour le traitement de l'image acquise par ladite sonde et pour le calcul de la position de l'aiguille par rapport à la prostate caractérisé en ce que le calculateur est commandé pour délivrer périodiquement une information sur le bougé de la prostate après chacune desdites étapes d'insertion afin de valider les paramètres d'insertion par rapport à un planning de traitement et le cas échéant modifier les paramètres d'insertion par rapport audit planning en cas de bougé supérieur à une valeur seuil.

Par « étape de pénétration » au sens du présent brevet, on entendra :
- une étape de déplacement de l'aiguille depuis une position externe à l'organe jusqu'à une position où la tête active est positionnée à l'intérieur de l'organe
- ainsi qu'une étape de déplacement de l'aiguille entre une première position où la tête active est positionnée à l'intérieur de l'organe jusqu'à une deuxième position à l'intérieur de l'organe.

Ladite modification des paramètres d'insertion consiste à modifier le planning.

Selon une variante, ladite modification des paramètres d'insertion consiste à modifier les paramètres d'insertion par un nouveau déplacement de l'aiguille.

Selon l'invention, les informations relatives au bougé de la prostate sont calculées par comparaison de l'image instantanée délivrée par la sonde rectale avec une image de référence de la prostate et par calcul d'une information quantitative des modifications entre lesdites images.

Selon une variante, on calcule la distance entre un point caractéristique de l'aiguille dans le référentiel instantané de la prostate et ledit point caractéristique de l'aiguille dans le référentiel de la prostate dans le planning, ladite distance étant ensuite comparée à ladite valeur-seuil pour valider conditionnellement le paramètre d'insertion.

Selon un mode de réalisation particulier, l'équipement selon l'invention comporte un support prolongé par une sonde rectale et comportant un porte-aiguille, l'équipement comportant des moyens pour déterminer l'orientation relative du porte-aiguille par rapport au volume d'acquisition de ladite sonde rectale.

Cette détermination de l'orientation du porte-aiguille par rapport au volume d'acquisition peut être réalisée par construction mécanique, par capteurs de position ou par traitement d'image.

De préférence, le porte-aiguille est motorisé et asservi par rapport à l'information de bougé, et comporte des moyens pour délivrer un signal représentatif de chaque nouvelle étape d'insertion.

Selon une variante particulière, le porte-aiguille est orientable pour modifier l'axe d'insertion en fonction dudit planning.

Selon un mode de réalisation particulier, l'équipement comporte un support solidaire de la sonde rectale, associé à un ensemble porte-aiguille articulé, constitué de deux tables primaires d'entraînement parallèles liées chacune à une table d'entraînement secondaire perpendiculaire, le porte-aiguille étant supporté par une traverse articulée en rotation par rapport auxdites tables d'entraînement secondaires, ladite traverse s'étendant perpendiculairement à l'axe de déplacement desdites tables secondaires et à l'axe de déplacement desdites tables primaires.

De préférence, le porte-aiguille de ce dernier mode de réalisation comporte un moyen de limitation d'effort débrayant mécaniquement l'entraînement du support de l'aiguille par rapport au porte-aiguille lorsque la résistance à l'insertion dépasse une valeur seuil correspondant à la situation de contact avec l'os pubien.

Selon une variante, l'équipement comporte un support lié d'une part à une sonde rectale et d'autre part à un moyen de positionnement du porte-aiguille.

L'invention sera mieux comprise à la lecture de la description qui suit, concernant un exemple non limitatif de mise en oeuvre de l'invention, se référant aux dessins annexés où :
- la figure 1 représente une vue schématique d'un équipement selon l'état de la technique
- la figure 2 représente une vue schématique d'un équipement selon l'invention
- la figure 3 représente un exemple de réalisation d'un robot porte-aiguille.
- la figure 4 représente une vue d'ensemble du robot porte-aiguille
- la figure 5 représente une vue détaillée du porte-aiguille.
- la figure 6 représente un diagramme d'utilisation d'une mise en oeuvre préférée de l'invention

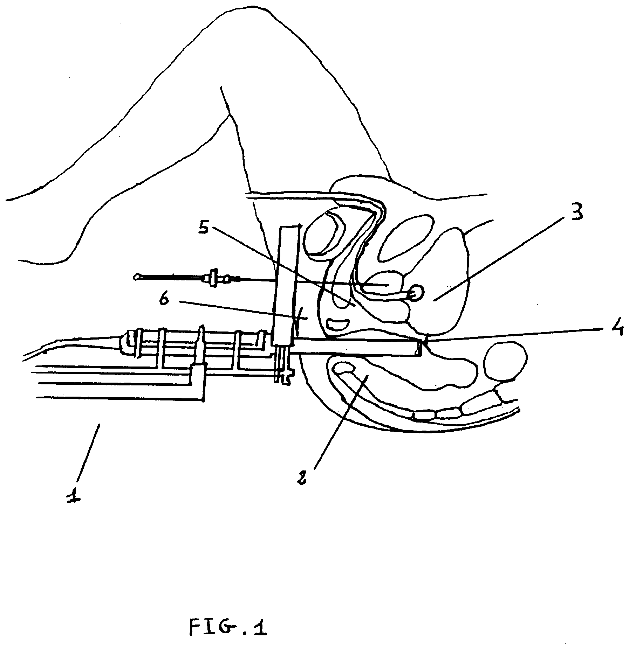

La figure 1 représente une vue schématique d'un équipement selon l'état de la technique.

Il comporte un support (1) sur lequel est fixé une sonde rectale (2) dont la tête est positionnée à proximité de la prostate (4) dont elle est séparée par la paroi périnéale. La tête comporte un moyen d'imagerie ultrasonore par balayage permettant de réaliser une image de la prostate pendant l'intervention.

Une aiguille (2) est manipulée par le chirurgien, aidé par un guide (6) facilitant le positionnement de l'aiguille (2), qui traverse la paroi du périnée pour pénétrer dans la prostate (4) selon une orientation et un planning prédéfini.

Cette aiguille peut être une aiguille creuse pour réaliser une série de biopsies selon un planning déterminé, permettant de détecter non seulement la présence d'une tumeur cancéreuse, mais aussi quelles zones de la prostate précisément présentent des proliférations cancéreuses.

Cette aiguille peut aussi être destinée à la curiethérapie « basse dose » de prostate, pour le traitement de la glande prostatique. Ce traitement consiste à implanter sous contrôle échographique des grains d'iode₁₂₅ au sein de la prostate.

Ces grains délivrent une irradiation continue durant environ un an. La prostate est ainsi traitée dans sa totalité à forte dose alors que les tissus « nobles » situés dans la prostate ou près de la prostate (tels que vessie, rectum, urètre) reçoivent une irradiation faible. Les aiguilles sont implantées dans la prostate, sous contrôle échographique, par voie trans-périnéale (elles traversent directement la peau du périnée entre l'anus et les bourses). Le nombre d'aiguilles et celui des grains dépendent du volume de la prostate. Lorsque les aiguilles sont correctement insérées dans la prostate, les grains radioactifs sont insérés à travers ces dernières, après quoi les aiguilles sont retirées.

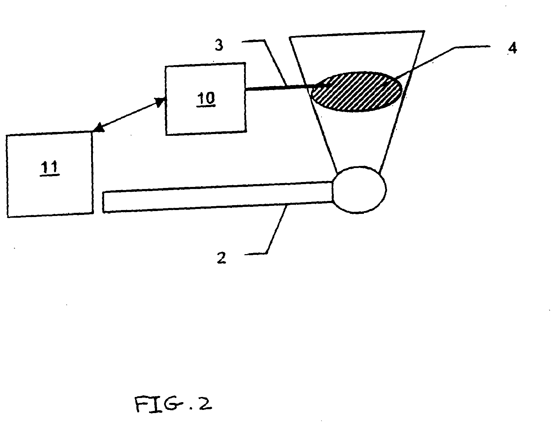

La figure 2 représente une vue schématique de l'équipement conforme à l'invention.

Le déplacement de l'aiguille (3) est commandé par un robot (10) motorisé dans l'exemple décrit. La sonde rectale (2) fournit une image ultrasonore 3D qui est exploitée par un calculateur (11). Ce calculateur exploite un logiciel de traitement périodique de l'image de la prostate (4) et de l'aiguille (3) par rapport à une image de référence.

Cette image de référence peut être une image invariable de la prostate acquise à un instant tₒ, ou une image rafraîchie périodiquement.

Le calculateur détermine des informations représentatives du bougé de la prostate, se traduisant par :
- un déplacement global de la prostate dans l'image acquise à un instant t, par rapport à l'image de référence
- une rotation globale de la prostate dans l'image acquise à un instant t, par rapport à l'image de référence
- une déformation de la prostate dans l'image acquise à un instant t, par rapport à l'image de référence.

En fonction de cette information représentative du bougé, le calculateur recalcule les paramètres d'insertion et modifie la commande du déplacement du robot (10).

Le but de ces traitements et de permettre une compensation des déformations induites mécaniquement par les contraintes exercées par l'aiguille, et indirectement par la sonde rectale, sur la prostate qui est un organe mou.

Le calculateur permet d'appliquer les traitements suivants :
1 - Calibration de la position relative de l'aiguille et de la sonde rectale
2 - Insertion de la sonde rectale et mise en place pour assurer l'acquisition d'une image de la prostate
3 - Acquisition d'une image tridimensionnelle I₀ de référence avant la première insertion de l'aiguille
4 - Enregistrement du planning
5 - Premier déplacement de l'aiguille selon la première étape du planning
6 - Acquisition d'une nouvelle image tridimensionnelle Iₙ correspondant à la nouvelle position de l'aiguille
7 - Calcul de la transformation spatiale entre l'image Iₙ et l'image initiale I₀ ou l'image précédente Iₙ₋₁.
8 - Détermination sur l'image Iₙ de la position de la tête de l'aiguille par rapport à la cible enregistrée sur le planning
9a) Si la distance entre la tête de l'aiguille est ladite cible est inférieure à une valeur-seuil, l'aiguille est retirée et on procède à un nouveau cycle d'insertion à partir de l'étape 5 susvisée
9b) Si la distance entre la tête de l'aiguille est ladite cible est supérieure à ladite valeur-seuil, vérification si la cible peut-être atteinte par un déplacement additionnel de l'aiguille
9b1) Dans l'affirmative, on procède à un déplacement supplémentaire de l'aiguille selon un planning actualisé et on revient à l'étape 8 susvisée
9b2) Dans la négative, on procède au retrait de l'aiguille et on recalcule le planning pour tenir compte du bougé de la prostate et on revient à l'étape 5 susvisée, pour la cible non atteinte.

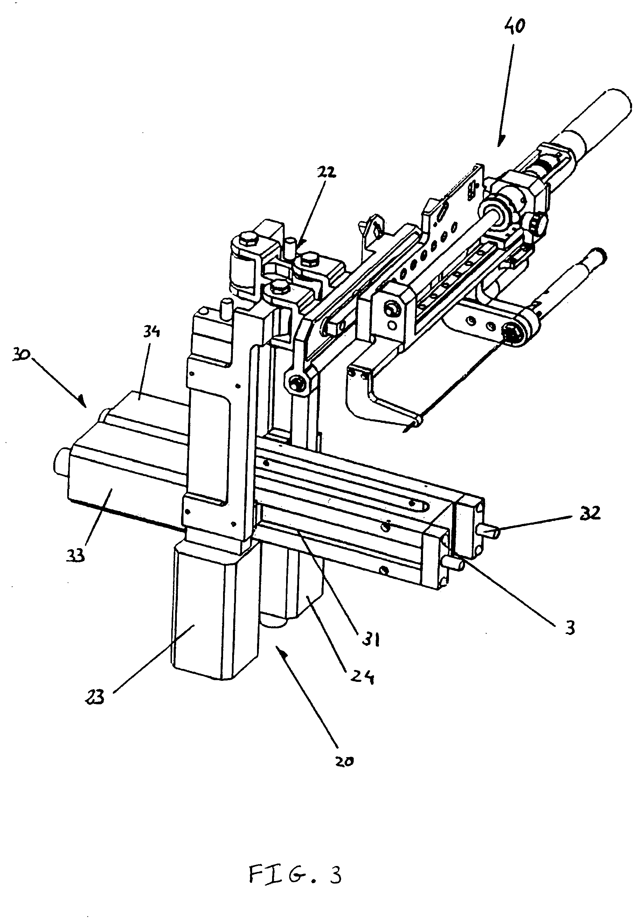

La figure 3 représente un exemple de réalisation d'un robot porte-aiguille.

Il est constitué par un module de positionnement formé de deux cadres (20, 30) perpendiculaires constituant des parallélogrammes déformables. Le mécanisme assurant le déplacement longitudinal de l'aiguille est constitué par un bras (40) articulé par rapport au premier cadre (20). Les axes de déplacement en translation des deux cadres (20, 30) et du bras (40) sont perpendiculaires et forme un référentiel à trois dimensions.

Le premier cadre (20) comprend deux rails parallèles (21, 22) et deux moteurs (23, 24). Le bras (40) est articulé par rapport à chacun de ces rails (21, 22) par des pivots permettant un basculement du bras porte-aiguille (40) dans un plan parallèle au plan passant par les deux axes médians des rails (21, 22).

Ce plan peut lui-même être orienté angulairement par une action du deuxième cadre (30). Ce deuxième cadre (30) comprend également deux rails (31, 32) et deux moteurs (33, 34) .

Chaque rail (31, 32) est articulé par un pivot à l'un des rails respectivement (21, 22) du premier cadre (20). En modifiant l'extension relative d'un des rails (31) par rapport à l'autre rail parallèle (32), on provoque une rotation du plan du premier cadre, autour d'un axe perpendiculaire à un plan passant par les rails (31, 32) du second cadre (30).

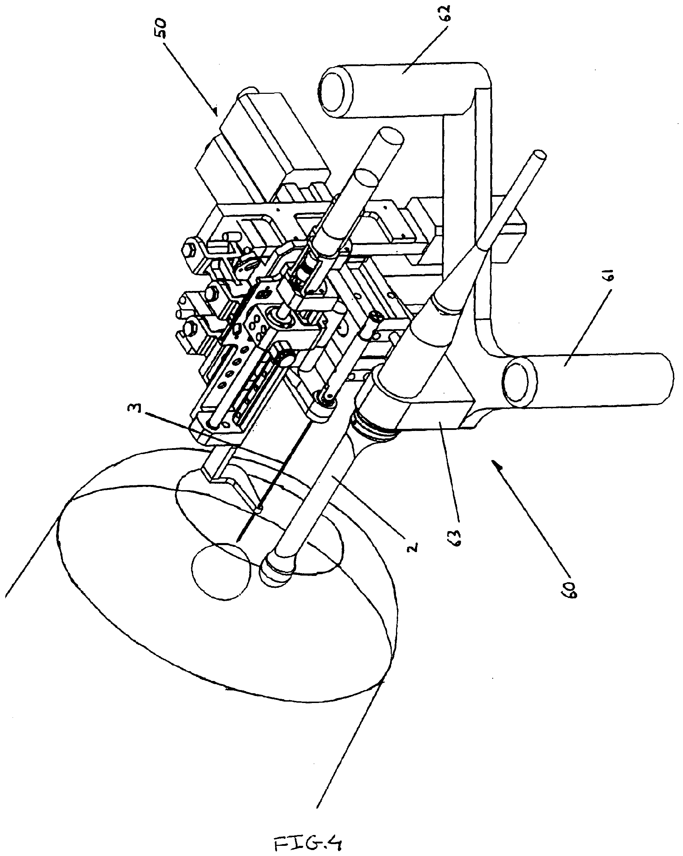

La figure 4 représente une vue d'ensemble du robot porte-aiguille.

Le module de positionnement (50) est monté sur un support (60) présentant deux bras (61, 62) destiné à la manipulation du robot par le chirurgien. Ce support comprend également un prolongement (63) pour la fixation de la sonde rectale (2) . Le prolongement (63) peut optionnellement être articulé pour permettre une modification de l'angle formé entre l'axe de la sonde rectale (2), et un axe de référence du module de positionnement (50).

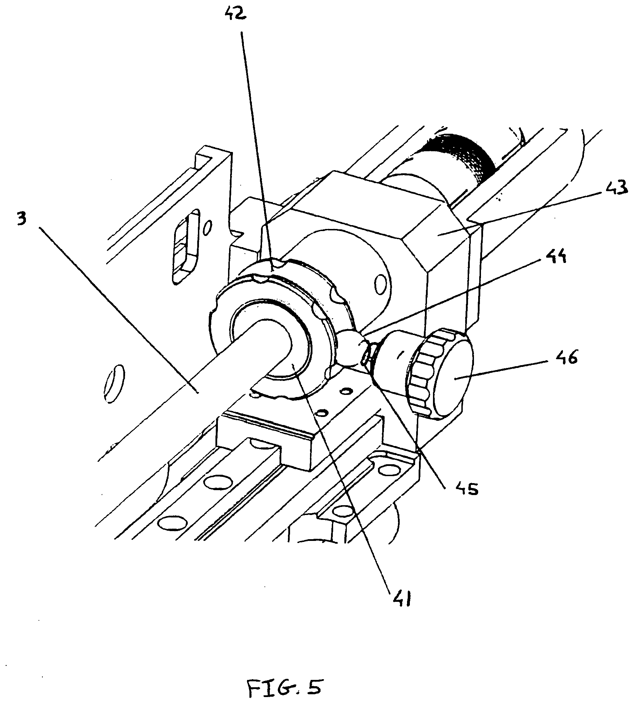

La figure 5 représente une vue détaillée du porte-aiguille.

L'aiguille (3) est solidaire d'un mandrin (41) de forme cylindrique, présentant une gorge (42). Ce mandrin (41) est engagé dans une cavité cylindrique d'une tête mobile (43) représentée partiellement, la partie avant étant transparente sur le dessin pour permettre la visualisation du mandrin.

La section de la cavité est sensiblement égale à la section extérieure du mandrin (41), afin de permettre un déplacement longitudinal sans effort entre le mandrin (41) et la tête mobile (43). Le couplage entre ces deux parties est assuré par un mécanisme à bille (44) comprenant un ressort (45) repoussant la bille (44) dans la cavité (42). Un bouton de réglage (46) permet d'ajuster le tarage du ressort (45).

Ce mécanisme assure un entraînement du mandrin par la tête mobile (43), avec une limitation de l'effort.

Lorsque la résistance rencontrée par l'aiguille dépasse une valeur seuil, la bille (44) est repoussée hors de la gorge (42) et le mandrin est alors désaccouplé de la tête mobile (43).

La valeur seuil est déterminée en fonction de la résistance rencontrée par l'aiguille lorsqu'elle bute par inadvertance sur l'os pubien.

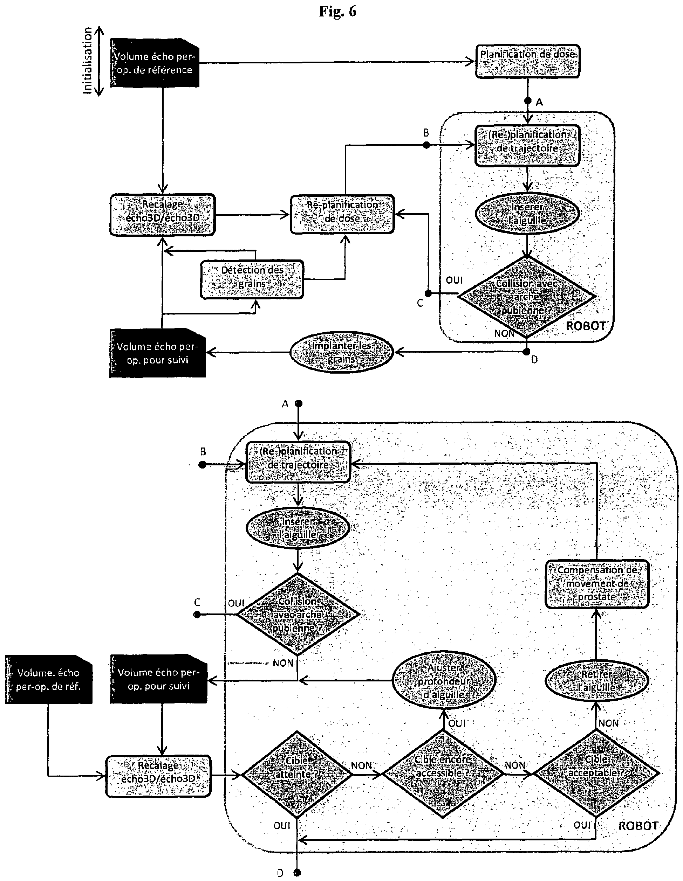

La figure 6 représente un diagramme d'utilisation clinique d'une mise en oeuvre préférée de l'invention.

Au début de la procédure, un volume échographique 3D de référence est acquis, sur lequel le contourage de la prostate et le planning de dose initial sont pratiqués. Dans cette phase de planning initial, les trajectoires d'aiguilles et les positions de grains sont définis par rapport à la prostate de référence extraite du volume échographique de référence. Par la suite, on applique pour chaque aiguille le processus suivant : la trajectoire d'aiguille est calculée dans le référentiel de coordonnées du robot grâce à une étape préliminaire de calibrage de la sonde échographique par rapport au robot. Le robot positionne l'aiguille à son point d'insertion au niveau du périnée, et l'insère. En cas d'interférence avec l'arche pubienne, l'aiguille est retirée et un planning partiel est à nouveau calculé afin de modifier la trajectoire de l'aiguille tout en conservant les contraintes de dose. Une fois que l'aiguille est insérée à la position planifiée, on applique une procédure de vérification afin de contrôler et de pallier tout déplacement ou déformation de la prostate induite par l'insertion de l'aiguille. Une fois le clinicien satisfait de la position finale de l'aiguille, les grains sont insérés tandis que l'aiguille est retirée progressivement. Un volume échographique 3D peut être acquis à ce moment afin de vérifier la position des grains de cette aiguille, globalement ou individuellement : un traitement d'image permet la localisation précise de la prostate et des grains, suite à quoi un nouveau planning peut s'avérer nécessaire pour prendre en compte des imprécisions persistantes, dans le positionnement final des grains par exemple. Au cours des étapes de re-planification, des modèles biomécaniques pourraient avantageusement être mis à contribution pour mieux prédire les bougés des organes. Il est important de noter que dans le schéma de contrôle présenté, le référentiel du plan de dosimétrie est lié à la prostate mobile, plutôt qu'à la sonde échographique fixe comme c'est le cas dans la procédure conventionnelle. Le fait d'acquérir un deuxième volume échographique et de le recaler au volume de référence permet de déformer le plan de dosimétrie en accord avec les bougés de la prostate et par là de vérifier que l'aiguille se trouve dans la position attendue dans la prostate. Dans le cas contraire, l'utilisateur ou le système vérifie si la cible déformée est toujours accessible via la même trajectoire d'aiguille, auquel cas la profondeur d'aiguille est ajustée. Alternativement, si le clinicien juge inacceptable la position courante de l'aiguille, celle-ci est retirée et un planning partiel est à nouveau calculé afin de compenser les bougés de prostate avant insertion de l'aiguille.

## Revendications

1. Equipement porte-aiguille (40) pour un traitement de la prostate impliquant une succession d'étapes d'insertion d'une aiguille (3) dans la prostate, comportant une sonde rectale (2) d'imagerie 3D ultrasonore et un calculateur pour le traitement de l'image acquise par ladite sonde et pour le calcul de la position de l'aiguille (3) par rapport à la prostate, le calculateur étant apte à délivrer périodiquement une information quantitative sur le bougé de la prostate après chacune desdites étapes d'insertion afin de valider les paramètres d'insertion par rapport à un planning de traitement après son adaptation à la forme et à la position à l'instant t de la prostate en déterminant sur l'image acquise la position de l'aiguille par rapport à une position cible de l'aiguille dans le planning en utilisant l'information quantitative délivrée et le cas échéant modifier les paramètres d'insertion par rapport audit planning en cas de distance entre la position de l'aiguille et ladite position cible supérieure à une valeur seuil, l'information quantitative relative au bougé de la prostate étant au moins l'un parmi un déplacement global de la prostate dans l'image acquise à un instant t, par rapport à l'image de référence, une rotation globale de la prostate dans l'image acquise à un instant t, par rapport à l'image de référence et une déformation de la prostate dans l'image acquise à un instant t, par rapport à l'image de référence et, l'information quantitative étant calculée par un traitement d'image comparant l'image instantanée délivrée à l'instant t par la sonde rectale (2) avec une image de référence de la prostate.

2. Equipement selon la revendication 1 **caractérisé en ce que** le calculateur est apte, en cas de distance entre la position de l'aiguille et ladite position cible supérieure à la valeur seuil, à déterminer si la position cible peut être atteinte par un déplacement additionnel de l'aiguille et dans la négative, à modifier les paramètres d'insertion en modifiant le planning.

3. Equipement selon la revendication 1 **caractérisé en ce que** le calculateur est apte, en cas de distance entre la position de l'aiguille et ladite position cible supérieure à la valeur seuil, à déterminer si la position cible peut être atteinte par un déplacement additionnel de l'aiguille et dans l'affirmative, ladite modification des paramètres d'insertion consiste à procéder au déplacement additionnel de l'aiguille (3) .

4. Equipement selon l'une quelconque des revendications précédentes **caractérisé en ce que** le calculateur est apte à calculer la distance entre un point caractéristique de l'aiguille (3) dans le référentiel instantané de la prostate et ledit point caractéristique de l'aiguille (3) dans le référentiel de la prostate dans le planning, et à ensuite comparer ladite distance à ladite valeur-seuil pour valider conditionnellement le paramètre d'insertion.

5. Equipement selon l'une quelconque des revendications précédentes **caractérisé en ce qu'**il comporte un support prolongé par une sonde rectale (2) et comportant un porte-aiguille (40), l'équipement comportant des moyens pour déterminer l'orientation relative du porte-aiguille (40) par rapport au volume d'acquisition de ladite sonde rectale (2).

6. Equipement selon l'une quelconque des revendications précédentes **caractérisé en ce que** ledit porte-aiguille (40) est motorisé et asservi par rapport à l'information de bougé, et comporte des moyens pour délivrer un signal représentatif de chaque nouvelle étape d'insertion.

7. Equipement selon la revendication précédente **caractérisé en ce que** ledit porte-aiguille (40) est orientable pour modifier l'axe d'insertion en fonction dudit planning.

8. Equipement selon l'une quelconque des revendications précédentes **caractérisé en ce qu'**il comporte support solidaire de la sonde rectale (2), associé à un ensemble porte-aiguille (40) articulé, constitué de deux tables primaires d'entraînement parallèles liées chacune à une table d'entraînement secondaire perpendiculaire, le porte-aiguille (40) étant supporté par une traverse articulée en rotation par rapport auxdites tables d'entraînement secondaires, ladite traverse s'étendant perpendiculairement à l'axe de déplacement desdites tables secondaires et à l'axe de déplacement desdites tables primaires.

9. Equipement selon la revendication 8 **caractérisé en ce que** le porte-aiguille (40) comporte un moyen de limitation d'effort débraillant l'entraînement du support de l'aiguille (3) par rapport au porte-aiguille (40) lorsque la résistance à l'insertion dépasse une valeur seuil correspondant à la situation de contact avec l'os pubien.

10. Equipement selon la revendication 1 **caractérisé en ce qu'**il comporte un support lié d'une part à une sonde rectale (2) et d'autre part à un moyen de positionnement du porte-aiguille (40) (grille).

## Patentansprüche

1. Nadelträger-Ausrüstung (40) für eine Behandlung der Prostata, die eine Abfolge von Einführschritten einer Nadel (3) in die Prostata impliziert, aufweisend eine rektale bildgebende 3D-Ultraschall-Sonde (2) und einen Rechner für die Verarbeitung des von der Sonde aufgenommenen Bildes und für die Berechnung der Position der Nadel (3) in Bezug auf die Prostata, wobei der Rechner imstande ist, periodisch eine quantitative Information über das Verwackeln der Prostata nach jedem der Einführschritte bereitzustellen, um die Einführparameter in Bezug auf eine Behandlungsplanung nach seiner Anpassung an die Form und an die Position der Prostata zum Zeitpunkt t durch Bestimmen, auf dem aufgenommenen Bild, der Position der Nadel in Bezug auf eine Zielposition der Nadel in der Planung durch Nutzung der bereitgestellten quantitativen Information zu bestätigen und bei einer Distanz zwischen der Position der Nadel und der Zielposition über einem Grenzwert die Einführparameter in Bezug auf die Planung gegebenenfalls zu ändern, wobei die quantitative Information bezüglich des Verwackelns der Prostata mindestens eine von einer allgemeinen Verlagerung der Prostata in dem aufgenommenen Bild zu einem Zeitpunkt t in Bezug auf das Referenzbild, einer allgemeinen Rotation der Prostata in dem aufgenommenen Bild zu einem Zeitpunkt t in Bezug auf das Referenzbild und einer Verformung der Prostata in dem aufgenommenen Bild zu einem Zeitpunkt t in Bezug auf das Referenzbild ist und die quantitative Information durch eine Verarbeitung des Bildes berechnet wird, bei der das momentane, zum Zeitpunkt t von der rektalen Sonde (2) bereitgestellte Bild mit einem Referenzbild der Prostata verglichen wird.

2. Ausrüstung nach Anspruch 1, **dadurch gekennzeichnet, dass** der Rechner imstande ist, bei einer Distanz zwischen der Position der Nadel und der Zielposition über einem Grenzwert zu bestimmen, ob die Zielposition durch eine zusätzliche Verlagerung der Nadel erreichbar ist und wenn nicht, die Einführparameter durch Ändern der Planung zu ändern.

3. Ausrüstung nach Anspruch 1, **dadurch gekennzeichnet, dass** der Rechner imstande ist, bei einer Distanz zwischen der Position der Nadel und der Zielposition über dem Grenzwert zu bestimmen, ob die Zielposition durch eine zusätzliche Verlagerung der Nadel erreichbar ist und wenn ja, die Änderung der Einführparameter darin besteht, die zusätzliche Verlagerung der Nadel (3) durchzuführen.

4. Ausrüstung nach einem der vorangehenden Ansprüche, **dadurch gekennzeichnet, dass** der Rechner imstande ist, die Distanz zwischen einem charakteristischen Punkt der Nadel (3) in dem momentanen Bezugssystem der Prostata und dem charakteristischen Punkt der Nadel (3) im Bezugssystem der Prostata in der Planung zu berechnen und danach die Distanz mit dem Grenzwert zu vergleichen, um den Einführparameter bedingt zu bestätigen.

5. Ausrüstung nach einem der vorangehenden Ansprüche, **dadurch gekennzeichnet, dass** sie einen Halter aufweist, der von einer rektalen Sonde (2) verlängert wird und einen Nadelträger (40) aufweist, wobei die Ausrüstung Mittel aufweist, um die relative Ausrichtung des Nadelträgers (40) in Bezug auf das Aufnahmevolumen der rektalen Sonde (2) zu bestimmen.

6. Ausrüstung nach einem der vorangehenden Ansprüche, **dadurch gekennzeichnet, dass** der Nadelträger (40) motorisiert ist und in Bezug auf die Verwackelungsinformation gesteuert wird und Mittel aufweist, um ein Signal bereitzustellen, das für jeden neuen Einführschritt repräsentativ ist.

7. Ausrüstung nach dem vorangehenden Anspruch, **dadurch gekennzeichnet, dass** der Nadelträger (40) ausrichtbar ist, um die Einführachse in Abhängigkeit von der Planung zu ändern.

8. Ausrüstung nach einem der vorangehenden Ansprüche, **dadurch gekennzeichnet, dass** sie einen einer angelenkten Nadelträgereinheit (40), gebildet von zwei parallelen primären Antriebstischen, die jeweils mit einem senkrechten sekundären Antriebstisch verbunden sind, zugeordneten Halter, der mit der rektalen Sonde (2) fest verbunden ist, aufweist, wobei der Nadelträger (40) von einem in Bezug auf die sekundären Antriebstische rotierend angelenkten Querträger gehalten wird, wobei sich der Querträger senkrecht zur Verlagerungsachse der sekundären Tische und zur Verlagerungsachse der primären Tische erstreckt.

9. Ausrüstung nach Anspruch 8, **dadurch gekennzeichnet, dass** der Nadelträger (40) ein Kraftbegrenzungsmittel aufweist, das den Antrieb des Halters der Nadel (3) in Bezug auf den Nadelträger (40) löst, wenn der Widerstand beim Einführen einen Grenzwert überschreitet, welcher der Kontaktsituation mit dem Schambein entspricht.

10. Ausrüstung nach Anspruch 1, **dadurch gekennzeichnet, dass** sie einen Halter aufweist, der zum einen mit einer rektalen Sonde (2) und zum anderen mit einem Positionierungsmittel des Nadelträgers (40) (Gitter) verbunden ist.

## Claims

1. Needle-carrying equipment (40) for a treatment of the prostate involving a succession of steps of inserting a needle (3) in the prostate, comprising an ultrasonic 3D imaging rectal probe (2) and a computer for the processing of the image acquired by said probe and to calculate the position of the needle (3) with respect to the prostate, the computer being capable of periodically delivering an item of quantitative information on the movement of the prostate after each of said insertion steps, in order to validate the insertion parameters with respect to a treatment plan after the adaptation thereof to the form and to the position at the time t of the prostate by determining on the acquired image, the position of the needle with respect to a target position of the needle in the plan by using the quantitative information delivered and, if necessary, modifying the insertion parameters with respect to said plan in case of distance between the position of the needle and said target position, greater than a threshold value, the quantitative information relating to the movement of the prostate being at least one from among an overall displacement of the prostate in the acquired image at a time t, with respect to the reference image, an overall rotation of the prostate in the acquired image at a time t, with respect to the reference image and a deformation of the prostate in the acquired image at a time t, with respect to the reference image and, the quantitative information being calculated by an image processing comparing the instantaneous image delivered at the time t by the rectal probe (2) with a reference image of the prostate.

2. Equipment according to claim 1, **characterised in that** the computer is capable, in case of distance between the position of the needle and said target position greater than the threshold value, of determining if the target position can be reached by an additional displacement of the needle and if not, of modifying the insertion parameters by modifying the plan.

3. Equipment according to claim 1, **characterised in that** the computer is capable, in case of distance between the position of the needle and said target position greater than the threshold value, of determining if the target position can be reached by an additional displacement of the needle and if so, said modification of the insertion parameters consists of proceeding with the additional displacement of the needle (3).

4. Equipment according to any one of the preceding claims, **characterised in that** the computer is capable of calculating the distance between a characteristic point of the needle (3) in the instantaneous frame of reference of the prostate and said characteristic point of the needle (3) in the frame of reference of the prostate in the plan, and of then comparing said distance to said threshold value to conditionally validate the insertion parameter.

5. Equipment according to any one of the preceding claims, **characterised in that** it comprises a support extended by a rectal probe (2) and comprising a needle carrier (40), the equipment comprising means to determine the relative orientation of the needle carrier (40) with respect to the acquisition volume of said rectal probe (2).

6. Equipment according to any one of the preceding claims, **characterised in that** said needle carrier (40) is motorised and enslaved with respect to the movement information, and comprises means to deliver a signal representative of each new insertion step.

7. Equipment according to the preceding claim, **characterised in that** said needle carrier (40) can be oriented to modify the insertion axis according to said plan.

8. Equipment according to any one of the preceding claims, **characterised in that** it comprises a support secured to the rectal probe (2), associated with a hinged needle carrying assembly (40), constituted of two parallel primary drive tables, each connected to a perpendicular secondary drive table, the needle carrier (40) being supported by a crosspiece hinged in rotation with respect to said secondary drive tables, said crosspiece extending perpendicularly to the displacement axis of said secondary tables and to the displacement axis of said primary tables.

9. Equipment according to claim 8, **characterised in that** the needle carrier (40) comprises a means for limiting effort, removing the drive from the support of the needle (3) with respect to the needle carrier (40) when the resistance to the insertion exceeds a threshold value corresponding to the location of contact with the pubic bone.

10. Equipment according to claim 1, **characterised in that** it comprises a support connected, on the one hand, to a rectal probe (2) and, on the other hand, to a means for positioning the needle carrier (40) (grid).
